(19) Europäisches Patentamt
European Patent Office
Office européen des brevets

(11) Veröffentlichungsnummer: **0 031 416**
**A1**

(12) **EUROPÄISCHE PATENTANMELDUNG**

(21) Anmeldenummer: **80106620.0**

(22) Anmeldetag: **29.10.80**

(51) Int. Cl.³: **C 07 C 45/00**, C 07 C 45/27, C 07 C 47/52, C 07 C 47/544, C 07 C 47/55, C 07 C 47/565, C 07 C 47/575, C 07 C 79/36, C 07 C 121/52

(30) Priorität: **29.11.79 DE 2948058**

(43) Veröffentlichungstag der Anmeldung: **08.07.81 Patentblatt 81/27**

(84) Benannte Vertragsstaaten: **AT BE CH DE FR GB IT LI LU NL SE**

(71) Anmelder: **DYNAMIT NOBEL AKTIENGESELLSCHAFT, Patentabteilung Postfach 1209, D-5210 Troisdorf, Bez. Köln (DE)**

(72) Erfinder: **Bernhardt, Günther, Dr., Rheinstrasse 33, D-5205 St. Augustin (DE)**
Erfinder: **Petersen, Egon-Norbert, Dr., Im Immenthal 42, D-5206 Neunkirchen-Seelscheid (DE)**
Erfinder: **Daum, Gerhard, Dr., Erkerstrasse 15c, D-5000 Köln 90 (DE)**

(54) **Verfahren zur Herstellung substituierter Benzaldehyde.**

(57) Die vorliegende Erfindung behandelt die Herstellung von kernsubstituierten Benzaldehyden aus den entsprechenden Benzylhalogeniden. Letztere werden durch organische Oxidationsmittel oxidiert. Erfindungsgemäß erfolgt die Oxidation mit Hilfe von Aminoxiden tertiärer Amino und in Anwesenheit von Wasser. Die Reaktionstemperaturen liegen zwischen 20 und 140° C. Das Wasser kann in Mengen von 0,1 bis 50 Vol.%, bezogen auf das Gesamtvolumen des Reaktionsgemisches, anwesend sein. Dies bedingt eine erhebliche Vereinfachung gegenüber dem Einsatz bekannter N-Aminoxide als Oxidationsmittel, die nur im wasserfreien Medium eingesetzt werden können. Die den Aminoxiden zugrundeliegenden Amine können aliphatischer, aromatischer oder heterocyclischer Natur sein.

EP 0 031 416 A1

0031416



Troisdorf, den 26.Nov.1979
OZ: 79067 (2993) Dr.Sk/Sch

DYNAMIT NOBEL AKTIENGESELLSCHAFT
Troisdorf, Bez. Köln

# Verfahren zur Herstellung substituierter Benzaldehyde

Gegenstand der vorliegenden Erfindung ist ein Verfahren zur technischen Herstellung von kernsubstituierten Benzaldehyden, bei dem die entsprechenden Benzylhalogenide zu dem Aldehyd oxidiert werden.

Es ist bekannt, die Oxidation von Benzylhalogeniden zu Benzaldehyden mit Hilfe organischer Oxidationsmittel durchzuführen. Der Nachteil dieser Verfahren liegt in den langen Reaktionszeiten und den geringen Ausbeuten. Außerdem bereitet die Aufarbeitung der Reaktionsprodukte oft große Schwierigkeiten.

Als organisches Oxidationsmittel wurde bereits Trimethylamin-N-oxid zur Umsetzung von unsubstituiertem Benzylbromid zu Benzaldehyd vorgeschlagen [Ber. 94 (1961), S. 1360]. Nachteilig bei dem dort beschriebenen Verfahren

ist der Einsatz eines großen Überschusses an Aminoxid, das im wasserfreien Zustand eingesetzt werden soll, indem es jedoch zu spontanem, explosionsartigem Zerfall neigt. Außerdem hat dieses Verfahren noch den Nachteil der geringen Ausbeute von unter 50 %.

Weiterhin ist es bekannt, als organisches Oxidationsmittel Pyridin-N-oxid bei der Oxidation von Benzylhalogeniden zu Benzaldehyden einzusetzen. [J.Org.Chem. 22 (1957), S. 1135] . Nachteilig bei dieser Arbeitsweise ist die Tatsache, daß das als Zwischenprodukt entstehende quaternäre Salz, gebildet aus Pyridinoxid und dem Benzylhalogenid stabil ist und erst durch eine zusätzliche Behandlung mit verdünnter Natronlauge in Benzaldehyd und Pyridin aufgespalten werden kann. Auch bei dieser Verfahrensweise, die ebenfalls mit wasserfreiem Pyridinoxid durchgeführt werden muß, liegen die Ausbeuten nur bei etwa 40 %.

Es bestand deshalb die Aufgabe, die Oxidation von Benzylhalogeniden zu Benzaldehyden technisch so durchzuführen, daß hohe Ausbeuten an reinem Produkt erhalten werden, wobei die Reaktion möglichst einstufig und mit leicht zugänglichen Verbindungen durchgeführt werden soll. Weiterhin soll sich das gesuchte Verfahren besonders auf die Herstellung von kernsubstituierten Benzaldehyden anwenden lassen, deren Herstellung im Falle einer o-Substitution von elektronenanziehenden Substituenten zusätzlich erschwert ist.

In Erfüllung dieser Aufgabe wurde nun ein Verfahren zur Herstellung von kernsubstituierten Benzaldehyden durch Oxidation der entsprechenden Benzhalogenide gefunden, das dadurch gekennzeichnet ist, daß man die Oxidation mit Aminoxiden tertiärer Amine bei Temperaturen zwischen 40

und 140 °C in Gegenwart von Wasser durchführt.

Bei dieser Verfahrensweise treten die Nachteile der bekannten Verfahren nicht oder nur in untergeordnetem Maße auf. Die Ausbeuten an Aldehyden sind erheblich höher als bei den bekannten Verfahren und werden dazu noch in einer wesentlich kürzeren Reaktionszeit erhalten. Diese hohen Ausbeuten sind unabhängig von der Art des Substituenten des Benzylhalogenids und der Reinheit dieses Stoffes. Es kann demzufolge auch ein Benzylhalogenid eingesetzt werden, das das entsprechende Benzalhalogenid und/oder das entsprechende Methylbenzol enthält.

Das erfindungsgemäße Verfahren hat weiterhin den Vorteil, daß als Ausgangsprodukte leicht herstellbare Verbindungen eingesetzt werden können. Dies gilt nicht nur für die Benzylhalogenide, sondern auch für die Aminoxide. Diese fallen bei ihrer Herstellung entweder als wässrige Lösungen oder in Hydratform an. Die Lösungen können direkt ohne besondere Reinigung eingesetzt werden.

Das bei der Umsetzung anwesende Wasser wird dem Reaktionsgemisch zweckmäßigerweise mit dem Aminoxid hinzugefügt. Dieses wird entweder in Form seines Hydrats oder als wässrige Lösung eingesetzt. Die dadurch in die Reaktionsmischung eingebrachte Wassermenge kann zwischen 0,1 bis 50 Vol.-% Wasser, bezogen auf das Gesamtvolumen des Reaktionsgemisches betragen. Vorzugsweise wählt man die hinzuzufügende Wassermenge aber so, daß der Gehalt an Wasser in dem Reaktionsgemisch zwischen 0,5 und 25 Vol.-% beträgt.

Die Reaktion des Benzylhalogenids mit dem Aminoxid läuft mit stöchiometrischen Mengen ab. Es genügt also, die Reaktionspartner im Äquivalentverhältnis 1 : 1 einzusetzen. Es ist jedoch empfehlenswert, das Aminoxid im geringen Überschuß anzuwenden, so daß pro Äquivalent Benzylhalogenid 1 bis 1,5 Äquivalente Aminoxid eingesetzt werden. Der Überschuß an Aminoxid kann jedoch auch größer sein; er sollte jedoch 2 Äquivalente pro Äquivalent Benzylhalogenid nicht überschreiten.

Die Reaktionstemperatur liegt im allgemeinen zwischen 20 und 140 °C. Bevorzugt wird jedoch im Bereich zwischen 40 und 100 °C gearbeitet. Es ist dabei empfehlenswert, die Umsetzung bei der Siedetemperatur des verwendeten Lösungsmittels durchzuführen, jedoch kann auch ein anderes, höher siedendes Lösungsmittel verwendet werden, sofern die angegebenen Temperaturgrenzen während der Umsetzung eingehalten werden.
Als Lösungsmittel und Reaktionsmedium eignen sich praktisch alle flüssigen Verbindungen, in denen die eingesetzten Benzylhalogenide löslich sind. Dazu gehören aliphatische, cycloaliphatische und aromatische Kohlenwasserstoffe, chlorierte Kohlenwasserstoffe, Alkylcyanide, Carbonsäureester, aliphatische Alkohole, nitrierte Kohlenwasserstoffe, bevorzugt Nitroaromaten und Gemische dieser Verbindungen. Es ist nicht notwendig, daß das organische Lösungsmittel mit Wasser mischbar ist. Selbst wenn Aminoxid und Benzylhalogenid in zwei verschiedenen Phasen gelöst sind, läuft die Reaktion in gewünschter Richtung ab. Selbstverständlich muß dabei für einen dauernden innigen Kontakt zwischen den zwei Phasen, z.B. durch Rühren, gesorgt werden.
Als Beispiel für einsetzbare Lösungsmittel seien genannt: Tetrachlorkohlenstoff, 1,2-Dichloräthan, Chloroform, Hexan, Cyclohexan, Toluol, Acetonitril, Essigsäuremethylester, Essigsäurebutylester, Essigsäureamylester, Propionsäureäthylester, Methanol, Äthanol, die Butanole, die Chlorbenzole und Nitrotoluole. Die Lösungsmittel sollen nach Möglichkeit einen Siedepunkt unter 150 °C besitzen.

Die Durchführung des erfindungsgemäßen Verfahrens erfolgt auf an sich bekannte Weise. Man kann entweder eine der beiden Reaktionskomponenten in einem geeigneten Reaktionsgefäß zusammen mit dem Reaktionsmedium vorlegen und die zweite Komponente im Verlauf der Reaktion zudosieren oder beide Komponenten gleichzeitig in das Reaktionsmedium eintragen. Zweckmäßigerweise wird das Aminoxid als wässrige Lösung zudosiert. Es ist empfehlenswert, vor dem Zudosieren einer oder beider Reaktionskomponenten den Inhalt des Reaktionsgefäßes auf die jeweils vorgesehene Reaktionstemperatur zu bringen. Anschließend wird das Reaktionsgemisch, z.B. durch Rühren, in guter Bewegung gehalten. Die Reaktion ist, je nach Konzentration der Reaktionsteilnehmer, nach 0,5 bis 3 Stunden beendet. Das Ende der Reaktion kann gaschromatographisch oder gravimetrisch (Reaktion des nicht umgesetzten Benzylhalogenids mit Pyridin) überprüft werden.

Wenn das Reaktionsmedium durch Wasser stark verdünnt ist, empfiehlt es sich, überschüssiges, d.h. für die Umsetzung nicht benötigtes Wasser aus dem Reaktionsraum zu entfernen. Diese Maßnahme soll vorzugsweise dann angewendet werden, wenn der Anteil des Wassers an dem gesamten Volumen mehr als 25 Vol.-% beträgt.

Die Entfernung des Wassers kann beispielsweise dadurch erfolgen, daß als Reaktionsmedium ein solches Lösungsmittel eingesetzt wird, das mit Wasser ein Azeotrop bildet wie z.B. Toluol, Tetrachlorkohlenstoff, Chloroform oder 1,2-Dichloräthan. Die Reaktion wird dann bei der Siedetemperatur dieses Azeotrops durchgeführt; man destilliert dann das Azeotrop ab und kann daraufhin gegebenenfalls das abgetrennte Lösungsmittel im Kreislauf wieder dem Reaktionsgemisch zuführen.

Es ist jedoch auch möglich, zu stark verdünnte wässrige Aminoxid-Lösungen vor ihrem Einsatz in dem erfindungsgemäßen Verfahren zu konzentrieren, indem man das Wasser unter reduziertem Druck oder durch azeotrope Destillation mit einem geeigneten Lösungsmittel bis auf die für die Reaktion gewünschte Konzentration entfernt oder geeignete Trocknungsmittel anwendet.

Die Aufarbeitung des Reaktionsgemisches erfolgt auf an sich bekannte Weise. Nach Abschluß der Umsetzung wird dem erhaltenen Reaktionsgemisch gegebenenfalls so viel Wasser hinzugefügt, daß eventuell ausgefallene Ammoniumsalze in Lösung gehen und die wässrige Phase von der organischen Phase getrennt werden kann. Die erhaltene organische Phase wird mit verdünnter Mineralsäure behandelt, anschließend gegebenenfalls mit verdünnten Basen neutralisiert und daraufhin mit Wasser gewaschen. Die Isolierung des Aldehyds erfolgt durch Abdestillation des Lösungsmittels. Zur Reindarstellung des Aldehyds kann eine Destillation oder Umkristallisation durchgeführt werden. Gegebenenfalls kann eine Zwischenisolierung und Reinigung des Aldehyds über die Bisulfit-Verbindung erfolgen.

Es ist weiterhin möglich, aus der bei der Aufarbeitung anfallenden wässrigen Ammoniumhalogenid-Lösung auf an sich bekannte Weise das Amin wiederzugewinnen, indem man diese Lösung mit Basen versetzt. Als Basen eignen sich bevorzugt die entsprechenden Alkali- und Erdalkaliverbindungen. Das auf diese Weise in Freiheit gesetzte Amin kann man dann nach seiner Isolierung wieder der Aminoxid-Herstellung zuführen.

Als Aminoxide tertiärer Amine können erfindungsgemäß die Aminoxide von aliphatischen, cycloaliphatischen, aromatischen oder heterocyclischen tertiären Aminen eingesetzt

werden. Auch die Aminoxide zweiwertiger tertiärer Amine sind einsetzbar. Die Gesamtzahl der Kohlenstoffatome der Aminoxide soll jedoch bevorzugt größer als drei sein, da sonst die Ausbeuten ohne zusätzliche weitere Maßnahmen zu gering sind.

Geeignete Aminoxide mit aliphatischen Reste, die gegebenenfalls substituiert sein können, sind z.B. Triäthylamin-N-oxid, Tripropylamin-N-oxid, Tributylamin-N-oxid, Isopropyldiäthylamin-N-oxid, Tri-(ß-äthoxiäthyl)-amin-N-oxid und ß-Diäthylamin-propionitril-N-oxid. Ein Aminoxid mit cycloaliphatischem Rest ist z.B. Dimethyl-cyclohexylamin-N-oxid. Das bevorzugte Aminoxid ist Triäthylamin-N-oxid.

Geeignete Aminoxide mit aromatischen Resten sind z.B. Dimethylanilin-N-oxid, Benzyldimethylanilin-N-oxid, p-Nitro-phenyldiäthylamin-N-oxid und p-N,N-Diäthylamino-benzonitril-N-oxid.

Geeignete heterocyclische Aminoxide sind beispielsweise N-alkyl- bzw. N-aryl-morpholin-N-oxide, wie N-Butylmorpholin-N-oxid, N-Phenyl-morpholin-N-oxid, N-aryl- oder -alkyl-tetra-hydrochinolin-N-oxid, N-aryl- oder -alkylpyrrolidin-N-oxide und deren Derivate, z.B. N-Methylpyroolidin-N-oxid und N-Phenylpyrrolidin-N-oxid.

Geeignete zweiwertige, tertiäre Aminoxide sind:
N,N,N',N'-Tetraäthyl-äthylendiamin-N,N'-dioxid, N,N'-Dimethyl-N,N'-dibenzyl-hexamethylendiamin-N,N'-dioxid und Triäthylendiamin-N,N'-dioxid.

Die als Ausgangsverbindungen einsetzbaren, im Benzolring ein- oder mehrfach substituierten Benzylhalogenide lassen sich durch folgende allgemeine Formel kennzeichnen:

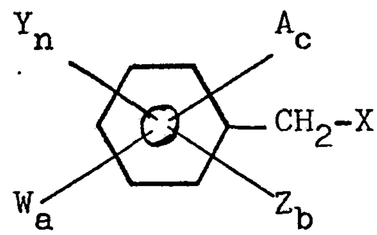

In dieser Formel bedeuten:

A = Nitro-Gruppen und/oder Nitril-Gruppen und/oder Acyloxi- und/oder Acylgruppen (c = 0 bis 2)

Y = Halogen, insbesondere Chlor und/oder Brom, und/oder Alkyl-Gruppen (n = 0 bis 5)

W = $-CH_2-X$ (a = 0 bis 5, vorzugsweise 0 bis 3)

Z = -OH und/oder Alkoxi- und/oder Phenoxi-Gruppen (b = 0 bis 3)

wobei a + b + n + c = 1 bis 5, vorzugsweise 1 bis 3 ist und X Chlor, Brom oder Jod bedeutet.

Der Rest A ist bevorzugt eine in o-Stellung stehende Nitrogruppe.

Im einzelnen seien als kernsubstituierte Benzylhalogenide genannt:

o,- m- oder p-Nitrobenzylbromid,
2,4-Dinitrobenzylbromid,
o-, m- oder p-Cyanobenzylbromid,
o-, m- oder p-Chlor- oder Brombenzylbromid,
Trichlor- oder Tribrombenzylbromid,
Tetrachlor- oder Tetrabrombenzylbromid,
Pentabrom- oder Pentachlorbenzylbromid,
o-, m- oder p-Phenoxibenzylbromid,
o-, m- oder p-Methylbenzylbromid,
2,4-Dimethylbenzylbromid,
2,4,6-Trimethylbenzylbromid,
o- oder m- oder p-Hydroxibenzylbromid,
o- oder m- oder p-Methoxibenzylbromid,
3,4,5-Trimethoxibenzylbromid

sowie die entsprechenden Chloride der genannten Verbindungen.

Weitere substituierte Bromide bzw. Chloride bzw. Jodide, die nach dem erfindungsgemäßen Verfahren umgesetzt werden können, sind solche, die einen weiteren $-CH_2X-$ -Substituenten in o-, m- oder p-Stellung aufweisen, wie z. B. o-, m- oder p-Xylylendibromid oder -chlorid, des weiteren auch diejenigen Verbindungen, die zusätzlich zu dem $-CH_2-X$-Substituenten einen oder mehrere Substituenten im Benzolkern aufweisen, z. B. Brom- oder Chlor- Substituenten, wie z. B. Tetrachlorxylylendibromid oder -chlorid, Tetrabrom-xylylendibromid- oder -chlorid, die entsprechenden Monochlor-, Dichlor- und Trichlorxylylendibromide sowie die entsprechenden -chloride.

Weiterhin können alkylsubstituierte Xylylendihalogenide, wie z. B. $C_1$ bis $C_2$-dialkylsubstituierte Xylylendihalogenide, wie Dimethyl- oder Diäthylxylylendihalogenide, dialkoxisubstituierte Xylylendihalogenide, wie Dimethoxi-xylylendihalogenide, sowie die entsprechenden hydroxisubstituierten Xylylendihalogenide, wie Mono- und Dihydroxi-xylylendihalogenide, umgesetzt werden.

Eine besonders vorteilhafte und verfahrenstechnische einfache Ausführung des erfindungsgemäßen Verfahrens besteht darin, daß die Rohhalogenierungsgemische von substituierten Toluolen, die gegebenenfalls neben dem entsprechenden Benzylhalogenid noch unverändertes Ausgangsmaterial oder Benzalhalogenid enthalten, mit Aminoxiden umgesetzt werden, und nach Abtrennung des Aldehyds das unveränderte substituierte Toluol in

den Halogenierungsprozeß zurückgeführt wird. So kann beispielsweise die Halogenierung des Toluols und die nachfolgende Umsetzung mit dem Aminoxid im selben Lösungsmittel und Reaktionsgefäß in einem Eintopf-Verfahren durchgeführt werden.

Bei der Herstellung von luftempfindlichen Aldehyden ist es von Vorteil, dem Reaktionsgemisch gegen Ende der Reaktion an sich bekannte Antioxidantien hinzuzugeben. Luftempfindliche Aldehyde sind z.B. solche, die positivierende Gruppen, wie z.B. eine oder mehrere Methylgruppen enthalten. Es genügen bereits Mengen zwischen 0,01 und 3,0 Gew.-%, bezogen auf das Reaktionsgemisch, um eine Autoxidation des Aldehyds zu verhindern. Als Antioxidantien eignen sich prinzipiell die bekannten Antioxidantien wie z.B. sterisch gehinderte Phenole. Als Beispiel sei 2,6 Di-tert.-butylphenol genannt.

Die gemäß der vorliegenden Erfindung herstellbaren Verbindungen sind wertvolle Zwischenprodukte für die Herstellung von Pharmazeutica, Herbiziden, Flammschutzmittel, Farbstoffen und optischen Aufhellern. Besonders der erfindungsgemäß erhältbare o-Nitrobenzaldehyd ist ein seit Jahren wichtiges Zwischenprodukt, dessen technische Herstellung bislang wegen der geringen Ausbeuten bei den bisher bekannten Verfahren nicht zufriedenstellend war.

Beispiel 1

In einem Dreihalskolben, der mit Rückflußkühler, Thermometer und Rührer versehen war, wurden 180 g wässriges Triäthylamin-N-oxid (75 %ig ≙ 1,15 Mol) und 1000 ml Tetrachlorkohlenstoff gegeben und im Verlaufe von 5 Minuten 216 g o-Nitrobenzylbromid (1,o Mol) unter guter Rührung eingetragen.

Die Mischung wurde 2 1/4 Stunden unter Rückfluß erhitzt und nach dem Abkühlen 100 ml Wasser hinzugefügt. Nach Abtrennen der wässrigen Phase wurde die Tetrachlorkohlenstoff-Lösung mit 100 ml 2N HCl, daraufhin dreimal mit 100 ml Wasser gewaschen, der Tetrachlorkohlenstoff abdestilliert und der Rückstand, der gemäß gaschromatographischer Prüfung bereits zu 97 % aus dem gewünschten Produkt besteht, einer Vakuumdestillation unterzogen.

Bei 0,4 mm gingen bei 84 bis 86° 128,2 g o-Nitrobenzaldehyd (≙ 85 % d. Th.) über (Fp: 40 - 41 °C).

Beispiele 2a bis 2d

Es wurde wie in Beispiel 1 gearbeitet, mit der Abänderung, daß anstelle von Tetrachlorkohlenstoff die in Tabelle 1 aufgeführten Lösungsmittel benutzt wurden:

Tabelle 1

| Beispiel | Lösungsmittel | o-Nitrobenzaldehyd Ausbeute [% d. Th.] | Fp [°C] |
|---|---|---|---|
| 2 a | Methanol | 73,7 | 40 - 41 |
| 2 b | Methylenchlorid | 80,5 | 41 - 42 |
| 2 c | Äthylendichlorid | 81,3 | 40 - 41 |
| 2 d | Acetonitril | 75,0 | 39 - 40,5 |

Beispiel 3

2639 g eines Bromierungsgemisches, bestehend aus 311 g o-Nitrobenzylbromid (1,44 Mol), 21 g o-Nitrobenzalbromid (0,007 Mol), 332 g o-Nitrotoluol (2,42 Mol) und 1975 g Tetrachlorkohlenstoff, wurden in einem Vierhalskolben, der mit Rührer, Thermometer, Tropftrichter, Rückflußkühler und Wasserabscheider versehen war, unter Rückfluß erhitzt. Innerhalb einer Stunde wurden 359,7 g einer 54 %igen wäßrigen Triäthylamin-N-oxid-Lösung zugetropft, wobei durch azeotrope Destillation 150 ml Wasser entfernt wurden. Anschließend wurde noch 1 1/2 h weiter am Rückfluß erhitzt.

Das ausgefallene Triäthylamin-hydrobromid wurde durch

die gerade dazu notwendige Menge an Wasser wieder in Lösung gebracht und nach dem Abkühlen die wäßrige Phase abgetrennt. Nach Waschen der organischen Phase mit verdünnter Salzsäure und Wasser wurde Tetrachlorkohlenstoff abdestilliert und der Rückstand im Vakuum fraktioniert destilliert. Bei $Kp_{0,4}$: 84 - 85°C gingen 174,4 g o-Nitrobenzaldehyd ≙ 80,2 % d. Th., bezogen auf eingesetztes o-Nitrobenzylbromid, über.

Das im Vorlauf übergegangene o-Nitrotoluol wog 315 g und entspricht 95 % der im Bromierungsgemisch enthaltenen Menge.

Die abgetrennte wäßrige Phase wurde in überschüssige verdünnte Natronlauge eingetropft und das freigesetzte Triäthylamin abdestilliert. Nach Trocknung und Reindestillation wurden 117,3 g ≙ 80 % d. Th. erhalten.

o-Nitrotoluol und Triäthylamin können der Bromierung bzw. der Aminoxid-Herstellung zugeführt werden.

Beispiel 4

In einem mit Rückflußkühler, Thermometer, Tropftrichter und Rührer versehenen Vierhalskolben wurden 108 g p-Nitrobenzylbromid (0,5 Mol) und 500 ml Tetrachlorkohlenstoff unter Rühren zum Sieden erhitzt und innerhalb von 30 Minuten 90,3 g 75 %iges, wäßriges Triäthylamin-N-oxid (≙ 0,58 Mol) zugetropft. Nach der Zugabe wurde der Rückfluß noch 2 h aufrechterhalten.

Nach Zugabe von 100 ml Wasser wurde vom ausgefallenen Niederschlag abgesaugt und zweimal mit 50 ml Wasser nachgewaschen. Von dem aus 2 Phasen bestehenden Fil-

trat wurde die organische Phase abgetrennt und diese mit 50 ml 2N HCl und daraufhin dreimal mit 50 ml Wasser gewaschen. Nach Trocknung über Natriumsulfat wurde der Tetrachlorkohlenstoff abgedampft und der Eindampfrückstand mit dem Filterkuchen vereinigt.

Die Gesamtausbeute an p-Nitrobenzaldehyd betrug 60,6 g, entsprechend einer Ausbeute von 80,2 % d. Th. (Fp: 103 - 104°C).

Beispiel 5

Es wurde wie in Beispiel 4 gearbeitet, mit der Abänderung, daß anstelle von Tetrachlorkohlenstoff als Lösungsmittel 400 ml Essigsäureäthylester benutzt wurde.

Die Ausbeute an p-Nitrobenzaldehyd betrug 71,0 % d. Th. (Fp: 102 - 104°C).

Beispiel 6

In einem mit Rührer, Rückflußkühler und Thermometer versehenen Dreihalskolben wurden 20,4 g 2,4,6-Tribrombenzylbromid (≙ 0,05 Mol), 50 ml 1,2-Dichloräthan und 9,4 g wäßriges Triäthylamin-N-oxid (75 %ig ≙ 0,06 Mol) während drei Stunden zum Sieden erhitzt.

Nach Zugabe von 30 ml Wasser wurde die organische Phase abgetrennt und mit 30 ml Wasser gewaschen. Nach Abdampfen des 1,2-Dichloräthans und Umkristallisieren des Rückstandes aus 55 ml Isopropanol wurden 14,1 g (82,0 % d. Th.) 2,4,6-Tribrombenzaldehyd vom Fp 97 - 98°C erhalten.

Elementaranalyse

| | C | H | Br |
|---|---|---|---|
| ber. | 24,89 | 0,87 | 69,94 |
| gef. | 25,01 | 0,78 | 69,70 |

Beispiel 7

In einem mit Rührer, Tropftrichter, Thermometer und Rückflußkühler ausgerüsteten Vierhalskolben wurden 58,0 g Tetrabromxylylendibromid (0,1 Mol) in 500 ml Toluol gelöst, bei 80 - 100°C 38,2 g wäßriges Triäthylamin-N-oxid (76,7 %ig ≙ 0,25 Mol) in 20 Min. zugetropft und die Reaktionsmischung noch 3 Stunden auf der obem angegebenen Temperatur gehalten.

Nach Zugabe von 100 ml Wasser wurde abgekühlt und das ausgefallene Produkt abgesaugt. Von dem aus zwei Phasen bestehenden Filtrat wurde die organische Phase abgetrennt und auf 100 ml eingeengt. Das beim Abkühlen ausgefallene Produkt wurde abgesaugt. Die vereinigten und getrockneten Filterrückstände ergaben 71,0 % d. Th. Tetrabromterephthaldialdehyd, Fp 226 - 232° C (aus Aceton).

Elementaranalyse

| | C | H | Br |
|---|---|---|---|
| ber. | 21,33 | 0,44 | 71,11 |
| gef. | 21,65 | 0,47 | 70,90 |

Beispiele 8a - 8n

Entsprechend Beispiel 1 wurde jeweils 1,0 Mol der in Tabelle 2 aufgeführten, im Benzolring substituierten

Benzylhalogenide in 1 l Tetrachlorkohlenstoff mit 180 g einer 75 %igen, wässrigen Triäthylamin-N-oxid-Lösung (1,15 Mol) zu den entsprechenden substituierten Benzaldehyden umgesetzt.

(Tabelle 2 siehe nächste Seite)

Tabelle 2

| Beispiel | subst. Benzylhalogenid | | Aldehyd | | | |
|---|---|---|---|---|---|---|
| | Chemische Bezeichnung | [g] | Ausbeute [g] | [% d.Th.] | Fp [°C] | Kp [mm/°C] |
| 8a | p-Brom-benzylbromid | 250,0 | 140,1 | 75,7 | 56-57 | - |
| 8b | o-Brom-benzylbromid | 250,0 | 145,1 | 78,4 | 21-22 | 230 |
| 8c | *Pentabrom-benzylbromid | 565,5 | 396,5 | 79,2 | 264-265 | |
| 8d | o-Chlor-benzylchlorid | 161,0 | 91,4 | 65,0 | - | 12/87-88 |
| 8e | 2,4-Dichlor-benzylbromid | 239,9 | 141,8 | 81,0 | 70-71 | |
| 8f | 2,6-Dichlor-benzylbromid | 239,9 | 148,8 | 85,0 | 70-71 | - |
| 8g | p-Cyano-benzylbromid | 196,1 | 106,6 | 81,3 | 100 | - |
| 8h | m-Phenoxibenzylbromid | 263,1 | 155,0 | 78,2 | - | 12/174-176 |
| 8i | p-Benzoyloxibenzylbromid | 291,2 | 181,2 | 80,1 | 72 | - |
| 8j | p-Hydroxi-benzylchlorid | 142,6 | 93,9 | 76,9 | 116 | - |
| 8k | 2,4-Dimethyl-benzylbromid | 199,1 | 110,7 | 82,5 | - | 12/101-102 |
| 8l | 2,4-Dinitro-benzylbromid | 261,0 | 158,9 | 81,0 | 72 | - |
| 8m | 3,4,5-Trimethoxibenzylchlorid | 216,7 | 163,0 | 83,1 | 76-77 | - |
| 8n | 2,4,6-Trimethylbenzylchlorid | 168,7 | 125,4 | 84,6 | - | 6/96-98 |

*Lösungsmittel Toluol

0031416

1 Beispiel 9

Entsprechend Beispiel 1 wurde 0,2 Mol o-Nitrobenzylbromid mit den in Tabelle 3 aufgeführten Aminoxiden umgesetzt.

Tabelle 3

| Beispiel | Aminoxid Chem.Bezeichnung | [Mol] | Lösungsmittel | o-Nitrobenzaldehyd Ausbeute [%d.Th.] | [Fp °C] |
|---|---|---|---|---|---|
| a | N,N,N',N'-Tetraäthylendiamin-N,N'-dioxid · $4H_2O$ | 0,12 | $CCl_4$ (200 ml) | 78,5 | 39,5-41,0 |
| b | N-Benzylpiperidin-N-oxid (80 %ige wäßr. Lsg.) | 0,23 | $CHCl_3$ (250 ml) | 80,2 | 41-42 |
| c | Dimethylanilin-N-oxid (72 %ige wäßr. Lsg.) | 0,22 | $CH_2Cl_2$ (200 ml) | 80,5 | 41-42 |
| d | Tripropylamin-N-oxid | 0,25 | $CCl_4$ (300 ml) | 84,8 | 41,5-42 |
| e | N-Äthylmorpholin-N-oxid | 0,24 | $CCl_4$ (200 ml) | 81,3 | 40-42 |

Vergleichsbeispiel

40 g Trimethylamin-N-oxid-dihydrat wurden in einer Destillationsapparatur in 250 ml Dimethylformamid gelöst und solange erhitzt, bis das übergehende Destillat eine Siedetemp. von 130 °C erreicht hatte.

Das restliche Lösungsmittel wurde an der Wasserstrahlpumpe abdestilliert, wobei das Heizbad bis auf 125 °C gebracht wurde.

Die Ausbeute an Trimethylamin-N-oxid betrug 24,5 g ≙ 90,7 % d. Th.

15 g des so erhaltenen, wasserfreien Trimethylaminoxids (0,2 Mol) wurden in 55 ml Chloroform gelöst und unter Eiskühlung innerhalb von 10 Min. 21,6 g o-Nitrobenzylbromid (0,1 Mol) eingetragen. Anschließend wurde noch 30 Min. am Rückfluß erhitzt. Die abgekühlte Reaktionslösung wurde mit 55 ml 2N HCl behandelt und anschließend mit verdünnter Natriumhydrogencarbonat-Lösung ausgeschüttet. Nach Trocknung über Natriumsulfat wurde das Chloroform unter reduziertem Druck abgedampft und der verbleibende Rückstand im Vakuum destilliert.

Bei $Kp_{0,4}$ 84 - 86° gingen 6,5 g o-Nitrobenzaldehyd ≙ 43 % der Theorie über.

0031416



Patentansprüche:

1. Verfahren zur Herstellung kernsubstituierter Benzaldehyde durch Oxidation von entsprechenden Benzylhalogeniden, dadurch gekennzeichnet, daß man die Oxidation in Gegenwart von Wasser mit Aminoxiden tertiärer Amine bei Temperaturen zwischen 20 und 140 °C durchführt.

2. Verfahren gemäß Anspruch 1, dadurch gekennzeichnet, daß man die Oxidation mit wässrigen Aminoxid-Lösungen durchführt.

3. Verfahren gemäß Anspruch 1 oder 2, dadurch gekennzeichnet, daß man die Oxidation in Gegenwart von 0,1 bis 50 Vol.-% Wasser, bezogen auf das Gesamtvolumen des Reaktionsgemischs, durchführt.

4. Verfahren gemäß einem der Ansprüche 1 bis 3, dadurch gekennzeichnet, daß pro Äquivalent Benzylhalogenid 1 bis 1,5 Äquivalent Aminoxid eingesetzt werden.

5. Verfahren gemäß einem der Ansprüche 1 bis 4, dadurch gekennzeichnet, daß man solche Aminoxide tertiärer Amine einsetzt, deren Gesamtkohlenstoffzahl größer als drei ist.

6. Verfahren gemäß einem der Ansprüche 1 bis 5, dadurch gekennzeichnet, daß man die Umsetzung in Gegenwart von organischen Lösungsmitteln durchführt.

7. Verfahren gemäß Anspruch 6, dadurch gekennzeichnet, daß als organisches Lösungsmittel halogenierte aromatische oder aliphatische Kohlenwasserstoffe eingesetzt werden.

8. Verfahren gemäß Anspruch 6 oder 7, dadurch gekennzeichnet, daß als organisches Lösungsmittel ein nitrierter, aromatischer oder aliphatischer Kohlenwasserstoff eingesetzt wird.

9. Verfahren gemäß einem der Ansprüche 1 bis 5, dadurch gekennzeichnet, daß man bei der Herstellung luftempfindlicher Aldehyde dem Reaktionsgemisch gegen Ende der Reaktion zusätzlich noch an sich bekannte Antioxidantien hinzufügt.

Europäisches Patentamt

# EUROPÄISCHER RECHERCHENBERICHT

0031416
Nummer der Anmeldung

EP 80 10 6620.0

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | DE - B - 1 152 696 (DEUTSCHE ADVANCE PRODUKTION)<br>* ganzes Dokument *<br>-- | 6 | C 07 C 45/00<br>C 07 C 45/27<br>C 07 C 47/52<br>C 07 C 47/544<br>C 07 C 47/55<br>C 07 C 47/565<br>C 07 C 47/575<br>C 07 C 79/36<br>C 07 C 121/52 |
| D | CHEMISCHE BERICHTE, Band 94, 1961 Weinheim<br>V. FRANZEN et al. " Eine neue Methode zur Darstellung von Carbonylverbindungen"<br>Seiten 1360 bis 1363<br>---- | | |
| | | | RECHERCHIERTE SACHGEBIETE (Int. Cl.³) |
| | | | C 07 C 45/00<br>C 07 C 45/27 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 02-04-1981 | PHILLIPS |